# EUROPÄISCHE PATENTANMELDUNG

(11) **EP 3 670 443 A1**
(43) Veröffentlichungstag der Anmeldung: **24.06.2020**
(21) Anmeldenummer: 19215478.9
(22) Anmeldetag: 12.12.2019
(51) Int. Cl.: C01B 3/16, C01B 3/38, C07C 1/20, C07C 29/151, C07C 41/09, C10G 2/00, C25B 1/04, F25J 3/02, C10K 3/02

(54) **VERFAHREN UND VORRICHTUNG ZUR HERSTELLUNG VON FLÜSSIGEM KRAFTSTOFF**

(30) Priorität: 20.12.2018 DE 102018222582; 10.01.2019 DE 102019200245
(71) Anmelder: Forschungszentrum Jülich GmbH, 52428 Jülich (DE)
(72) Erfinder: Schemme, Steffen, 47051 Duisburg (DE); Tschauder, Andreas, 52428 Jülich (DE); Samsun, Dr.-Ing., Remzi Can, 52511 Geilenkirchen (DE); Peters, Prof. Dr., Ralf, 52146 Würselen (DE); Stolten, Prof. Dr., Detlef, 52076 Aachen (DE)
(74) Vertreter: Gille Hrabal

(57) **Zusammenfassung**

Die Erfindung betrifft ein Verfahren zur Herstellung von Benzin mit den Schritten:
• Herstellung von Methanol aus Wasserstoff, Kohlendioxid und/ oder Kohlenmonoxid in einem ersten Reaktor;
• Herstellung von gasförmigen und flüssigen Kohlenwasserstoffen aus dem Methanol in einem zweiten Reaktor;
• Weiterleiten der gasförmigen und flüssigen Kohlenwasserstoffe aus dem Reaktor in eine Trennstation;
• Abtrennen von flüssigem Wasserstoff in der Trennstation und Herausführen des abgetrennten flüssigen Kraftstoffes aus der Trennstation;
• Weiterleiten von einem Nebenprodukt aus der Trennstation in einen Reformer;
• Herstellen von Synthesegas im Reformer aus dem Nebenprodukt.

## Beschreibung

Die Erfindung betrifft ein Verfahren und eine Vorrichtung zur Herstellung eines flüssigen Kraftstoffs, also ein flüssiges Gemisch aus verschiedenen Kohlenwasserstoffen. Solche flüssigen Kraftstoffe sind unter der Bezeichnung Benzin, Diesel oder Kerosin bekannt.

Wasserstoff (H₂) kann durch Elektrolyse aus Wasser (H₂O) gemäß folgender Gleichung erzeugt werden:

2 H₂O ↔ 2 H₂ + O₂.

Wasserstoff kann durch Reaktion mit Kohlendioxid (CO₂) in Methanol (CH₄O) katalytisch umgewandelt werden. Die Reaktion verläuft exotherm insbesondere wie folgt:

CO₂ + 3 H₂ ↔ CH₄O.

Weitere Reaktionen, die stattfinden können, verlaufen wie folgt:

CO₂ + H₂ ↔ CO + H₂O

CO + 2 H₂ ↔ CH₄O.

Aus Kohlendioxid und Wasserstoff kann also Kohlenmonoxid und Wasser hergestellt werden. Diese Reaktion wird umgekehrte Wassergas - Shift - Reaktion genannt.

Wasserstoff kann durch Reaktion mit Kohlenmonoxid (CO) in Methanol katalytisch umgewandelt werden.

Das Gasgemisch aus Wasserstoff und Kohlenmonoxid wird Synthesegas genannt. Synthesegas kann aus Kohle, Erdgas, Erdöl oder Biomasse hergestellt werden. Synthesegas kann durch Elektrolyse von CO₂ und H₂O erzeugt werden.

Methanol wird großtechnisch aus Synthesegas im Nieder- oder Mitteldruckverfahren hergestellt. Das Mitteldruckverfahren verwendet einen Druck von 100 bis 250 bar bei einem Temperaturbereich von 220 bis 300 °C. Das Niederdruckverfahren wird bei einem Druck von 50 bis 100 bar und Temperaturen zwischen 200 und 300 °C durchgeführt.

Für die Herstellung von Methanol aus Synthesegas und/oder aus Kohlendioxid und Wasserstoff werden Katalysatoren wie z. B. Zink-Chromoxid-Katalysatoren, kupferbasierte Katalysatoren mit Zusätzen von Zinkoxid und/oder Aluminiumoxid, Kombinationen aus Kupfer und Seltenerdmetallen; Kupferoxid/Zinkoxid-Katalysatoren, Kupferoxid/Zinkoxid/Chromoxid-Katalysatoren oder Kupfer-Zinkoxid-Aluminiumoxid Katalysatoren verwendet. Da die Methanolsynthese ein exothermer Prozess ist, sind geringe Temperaturen thermodynamisch günstig. Allerdings sind geeignete Katalysatoren erst ab Temperaturen von typischerweise ca. 250 °C ausreichend aktiv. So sind konventionelle Cu/ZnO/Al₂O₃-Katalysatoren erst ab etwa 230 °C aktiv und bis 290 °C temperaturstabil.

Es ist aus der Druckschrift "Aasberg-Petersen, K., et al., Recent developments in autothermal reforming and pre-reforming for synthesis gas production in GTL applications; Fuel Processing Technology, 2003. 83(1-3): p. 253-261 DOI: http://dx.d0i.0rg/10.1016/S0378-3820(03)00073-0" bekannt, durch autothermische Reformierung ein Synthesegas herzustellen.

Aus der Druckschrift DE 10 2009 046 790 A1 ist bekannt, aus einem Synthesegas Benzin herzustellen. Die katalytische Umwandlung von Methanol in Benzin wird auch Methanol-to Gasoline-Prozess oder abgekürzt MTG-Prozess genannt. Die Druckschrift US 4,523,046 (aus 1985) offenbart ein Verfahren zur Erzeugung von Benzin aus Methanol. Aus der Druckschrift "Phillips, S.D., et al., Gasoline from Wood via integrated Gasification, Synthesis, and Methanol-to-Gasoline Technologies. 2011, NREL - National Renewable Energy Laboratory: Golden, Colorado., p. 115" ist eine Synthese von Benzin aus Methanol bei 400 °C und 13 bar an ZSM-5-Zeolithkatalysatoren bekannt.

Ein Verfahren zur Herstellung von flüssigen Kraftstoffen aus kohlen- und wasserstoffhaltigen Ausgangsstoffen wie Kohle, Erdgas und Biomasse ist unter der Bezeichnung Fischer-Tropsch-Synthese bekannt. Zunächst kann beispielsweise durch Vergasung ein Synthesegas erzeugt werden, also ein Gasgemisch aus CO und H₂. In der Fischer-Tropsch-Synthese werden aus dem Synthesegas langkettige Kohlenwasserstoffe erzeugt. Das Synthesegas kann beispielsweise heterogenkatalytisch in gasförmige und flüssige Kohlenwasserstoffe bei Temperaturen von etwa 160 bis 300 °C und Drücken von etwa 25 bar umgewandelt werden. Auch höhere Drücke von beispielsweise 40 bar sind möglich. Als Katalysatoren können Kontakte auf Basis von Cobalt oder Eisen dienen.

Als Nebenprodukt fallen bei der Herstellung eines synthetischen flüssigen Kraftstoffs aus Methanol kürzere Kohlenwasserstoffe wie Methan, Ethan, Propan oder n-Butan an. Derartige Nebenprodukte werden in der Druckschrift "Buddenberg, T., C. Bergins, and S. Schmidt, Power to fuel as a sustainable business model for cross-sectorial energy storage in industry and power plants, in 5th Conference on Carbon Dioxide as Feedstock for Fuels, Chemistry und Polymers. 2016: Cologne, Germany" als LPG bezeichnet.

Aus der Dissertation "Daniel Helmut König, Techno-ökonomische Prozessbewertung der Herstellung synthetischen Flugturbinentreibstoffes aus CO2 und H2, Universität Stuttgart, 2016" ist bekannt, in einem ersten Schritt Wasserstoff durch Wasserelektrolyse zu erzeugen. Zusammen mit Kohlenstoffdioxid wird mit der reversen Wassergas-Shift-Reaktion Synthesegas gebildet, welches mittels Fischer-Tropsch-Synthese in langkettige Kohlenwasserstoffe umgesetzt wird. Eine nachfolgende Produktauftrennung und -aufbereitung liefert synthetischen Flugturbinentreibstoff und die Nebenprodukte Benzin und Diesel. Aus der Druckschrift ist außerdem bekannt, eine Hochtemperatur-Co-Elektrolyse einzusetzen, um aus Dampf und Kohlenstoffdioxid direkt Synthesegas zu produzieren.

Aus der Druckschrift US 2011/0152593 A1 ist ein Verfahren zur Herstellung von Kohlenwasserstoffen bekannt, um diese als Kraftstoff zu verwenden. Bei dem Verfahren kann eine Rückführung von Kohlenwasserstoffen, Kohlenmonoxid, Kohlendioxid und Wasserstoffgas vorgesehen sein.

Es ist Aufgabe der Erfindung, flüssigen Kraftstoff und zwar einerseits insbesondere Benzin oder andererseits Diesel und Kerosin verbessert herzustellen.

Die Aufgabe wird durch ein Verfahren mit den Merkmalen des Patentanspruchs 1 gelöst. Eine Vorrichtung umfasst zur Lösung der Aufgabe die Merkmale des Nebenanspruchs.

Zur Lösung der Aufgabe umfasst das Verfahren die folgenden Schritte:
- Einleiten von Kohlendioxid und Wasserstoff in einen ersten Reaktor;
- Zuführen von Kohlenmonoxid aus einem Reformer in den ersten Reaktor;
- Herstellen von Methanol aus dem Wasserstoff, Kohlendioxid und Kohlenmonoxid in dem ersten Reaktor mithilfe eines Katalysators und/oder Herstellen eines Synthesegases in dem ersten Reaktor mithilfe eines Katalysators und/oder Herstellen von Methylether in dem ersten Reaktor mithilfe eines Katalysators;
- Zuführen von Methanol und/oder Synthesegas und/oder Methylether aus dem ersten Reaktor in einen zweiten Reaktor;
- Herstellen von Kohlenwasserstoffen aus dem Methanol und/oder aus dem Synthesegas und/oder aus dem Dimethylether mithilfe des zweiten Reaktors;
- Zuführen der Kohlenwasserstoffe in eine Trennstation;
- Abtrennen von flüssigem Kraftstoff in der Trennstation und Herausführen des abgetrennten flüssigen Kraftstoffs aus der Trennstation;
- Zuführen eines Nebenprodukts aus der Trennstation in den Reformer;
- Herstellen des Kohlenmonoxids in dem Reformer aus dem Nebenprodukt.

Einleiten von Kohlendioxid und Wasserstoff in einen ersten Reaktor meint, dass extern bereitgestelltes Kohlendioxid und extern bereitgestellter Wasserstoff in den ersten Reaktor eingeleitet wird. Kohlendioxid wird beispielsweise durch Abscheidung aus Luft, Industrieabgasen oder aus bei einer Verbrennung entstehenden Gasen extern bereitgestellt. Wasserstoff wird beispielsweise durch Elektrolyse extern bereitgestellt.

Als Katalysator können im ersten Reaktor ein oder mehrere Katalysatoren eingesetzt sein, die für eine katalytische Umwandlung eines Gemisches aus CO₂, CO und H₂ in CH₄O und/oder in Synthesegas und/oder Dimethylether bekanntermaßen geeignet sind.

Um Stoffe im Sinne der vorliegenden Erfindung zuführen zu können, kann eine rohrförmige Verbindung vorgesehen sein, über die ein Fluid von einer Station zu einer nächsten Station geleitet werden kann. So kann beispielsweise eine rohrförmige Verbindung den Reformer mit dem ersten Reaktor so verbinden, dass Kohlenmonoxid aus dem Reformer herausgeleitet und das herausgeleitete Kohlenmonoxid in den ersten Reaktor hineingeleitet werden kann.

Als Katalysator können im zweiten Reaktor ein oder mehrere Katalysatoren eingesetzt sein, die für eine katalytische Umwandlung von Methanol in flüssigen Kraftstoff und/oder von Synthesegas in flüssigen Kraftstoff bekanntermaßen geeignet sind.

Im zweiten Reaktor kann aber auch zunächst Dimethylether hergestellt werden. Aus dem hergestellten Dimethylether können in einem weiteren Reaktor Olefine hergestellt werden, um hieraus schließlich gewünschte flüssige Kraftstoffe zu gewinnen. Bei der anschließenden Synthese von Olefinen zu flüssigen Kraftstoffen (Benzin, Diesel, Kerosin) fällt wieder LPG an. Auch dieses kann zu H₂/CO im Reformer reformiert werden, um es wieder erfindungsgemäß der Methanol- und/ oder Dimethylether-Synthese zuzuführen. Auch die beim Syntheseschritt von Olefinen zu Kerosin/Diesel/(Benzin) anfallenden Nebenprodukte können also reformiert und zur Methanol- bzw. Dimethylether- Synthese zurückgeführt werden. Im Prinzip können alle (gasförmigen) Nebenprodukte zu Edukten für die Methanol- bzw.- Dimethylether - Synthese reformiert werden.

Werden im zweiten Reaktor Kohlenwasserstoffe aus dem Methanol unmittelbar erzeugt, so werden die erzeugten Kohlenwasserstoffe aus dem zweiten Reaktor der Trennstation zugeführt. Wird im zweiten Reaktor zunächst Dimethylether erzeugt, um in einem nachfolgenden Verarbeitungsschritt Kohlenwasserstoffe aus dem Dimethylether herzustellen, dann werden die Kohlenwasserstoffe im Anschluss an den entsprechenden Verarbeitungsschritt der Trennstation zugeführt.

Die Trennstation ist so eingerichtet, dass ein der Trennstation zugeführtes Gemisch in Bestandteile getrennt werden kann. Die Trennstation kann eine Rektifikationskolonne umfassen, um Fraktionen mit unterschiedlichen Siedetemperaturen aus dem Gemisch abzutrennen. Die Trennstation kann eine Einrichtung umfassen, die Dichteunterschiede ausnutzt, um beispielsweise gasförmige Bestandteile von flüssigen Bestandteilen zu trennen. Die Trennstation kann einen Flüssigkeitsabscheider umfassen, um Gase von Flüssigkeiten zu trennen.

Insbesondere ist die Trennstation so eingerichtet, dass Benzin abgetrennt und das abgetrennte Benzin aus der Trennstation herausgeführt werden kann. Dies ist vor allem dann vorteilhaft, wenn Methanol in dem ersten Reaktor hergestellt wurde.

In den ersten Reaktor werden also insbesondere für die Herstellung von Benzin Kohlenmonoxid, Kohlendioxid und Wasserstoff eingeleitet, um dieses Gasgemisch im ersten Reaktor katalytisch in Methanol umzuwandeln. Es findet also eine Methanolsynthese aus H₂, CO₂ und CO statt, was sich positiv auf die Katalysatorlebensdauer auswirkt. Es entstehen bei der erfindungsgemäßen Methanolsynthese nur relativ geringe Mengen an Wasser als Koppelprodukt, was den Energieverbrauch von Pumpen, Verdichtern und Kolonnen gering hält. Insgesamt kann ein flüssiger Kraftstoff wie Benzin mit hohem Wirkungsgrad, hoher Ausbeute und geringem technischen Aufwand hergestellt werden.

Aus dem Nebenprodukt kann mit Hilfe des Reformers unter Einsatz von O₂ und H₂O das Kohlenmonoxid hergestellt werden.

Es kann durch autotherme Reformierung das Kohlenmonoxid aus dem Nebenprodukt hergestellt werden.

Als Nebenprodukt können Methan (CH₄), Ethan (C₂H₆), Propan (C₃H₈) und/oder Butan (C₄H₁₀) entstehen, die aus der Trennstation heraus dem Reformer zugeführt werden. Methan (CH₄), Ethan (C₂H₆), Propan (C₃H₈) und/oder Butan (C₄H₁₀) werden dann also von der Trennstation zu dem Reformer weitergeleitet. Alle Nebenprodukte, die nicht der Benzinfraktion entsprechen, können dem Reformer zugeführt werden. Von der Erfindung ist aber auch umfasst, dass die aus der Trennstation herausgeführten Nebenprodukte nur zum Teil in den Reformer eingeleitet werden.

Im Reformer können sich im Anschluss an eine Reformierung H₂, CO und/oder CO₂ befinden, die aus dem Reformer herausgeleitet und in den ersten Reaktor hineingeleitet werden. Es kann außerdem aus thermodynamischen Gründen CH₄ entstehen. Auch CH₄ kann in den ersten Reaktor hineingeleitet werden. CH₄ ist aber grundsätzlich nicht erwünscht.

Wasserdampf, der in dem ersten oder zweiten Reaktor entsteht, kann in die Trennstation für die Durchführung der Trennung und/oder in den Reformer für die Durchführung der Reformierung eingeleitet werden, um die Produktivität weiter zu verbessern.

Aus dem ersten Reaktor kann Gas periodisch herausgeführt werden, was sich ebenfalls günstig auf die Produktivität auswirken kann. Herausführen meint, dass dieses Gas nicht in den zweiten Reaktor eingeleitet wird. Es wird auf diese Weise beispielsweise 10 Vol.-% an Gas, welches in den ersten Reaktor eingeleitet wurde, nicht in den zweiten Reaktor hineingeleitet, sondern stattdessen auf andere Weise weiter verwertet.

Innerhalb der Methanolsynthese wird nicht umgesetztes Edukt (H₂, CO₂, CO) vom Reaktorprodukt vorteilhaft abgeschieden und innerhalb des Verfahrens zurückgeführt. Übrig bleibt ein Gemisch aus Methanol und Wasser. Dieses kann in einer Trennkolonne in Methanol und Wasser getrennt werden. So kann reines Methanol aus dem Prozess austreten.

Es können also eine oder mehrere weitere Trennstationen vorhanden sein, um Produkte geeignet zu separieren. So kann beispielsweise eine Rektifikationskolonne als Trennstation vorgesehen sein, die das im ersten Reaktor erzeugte Methanol von anderen Bestandteilen trennt, um so vorteilhaft eine hohe Reinheit von mehr als 90 Gew.-%, so zum Beispiel eine Reinheit von wenigstens 99,9 Gew.-%, zu erhalten. Erst im Anschluss daran wird das Methanol dem zweiten Reaktor zugeführt.

Abwärme, die aufgrund der durchgeführten exothermen Reaktionen zur Verfügung steht, kann vorteilhaft ein oder mehreren Rektifikationskolonnen oder Destillationskolonnen zugeführt und so für ein Trennen genutzt werden. Abwärme kann auch bei der Eduktbereitstellung genutzt werden.

In einer vorteilhaften Ausgestaltung des Verfahrens werden aus der Trennstation herausgeführte Nebenprodukte teilweise in den Reformer eingeleitet und teilweise in den ersten Reaktor. Durch diese Ausgestaltung kann der Energiebedarf für die Herstellung gesenkt werden und zwar insbesondere der Bedarf an elektrischer Energie. Diese Ausgestaltung ist vor allem für den Fall günstig, dass Diesel, Kerosin und/oder in dem ersten Reaktor ein Synthesegas erzeugt wird. Elektrische Energie kann vor allem deshalb eingespart werden, weil der Wasserstoffbedarf gering gehalten werden kann und für die Erzeugung von Wasserstoff in der Regel elektrische Energie eingesetzt wird.

Vorzugsweise werden aus der Trennstation herausgeführte Nebenprodukte zum überwiegenden Teil in den Reformer eingeleitet. Ein geringerer Teil wird in den ersten Reaktor eingeleitet, um weiter verbessert den Bedarf an elektrischer Energie gering zu halten. Vorzugsweise werden aus der Trennstation herausgeführte Nebenprodukte zu wenigstens 60 Vol.-%, besonders bevorzugt zu wenigstens 70 Vol.-%, in den Reformer eingeleitet. Vorzugsweise werden aus der Trennstation herausgeführte Nebenprodukte zu wenigstens 10 Vol.-%, besonders bevorzugt zu wenigstens 20 Vol.-%, in den ersten Reaktor eingeleitet. Aus der Trennstation herausgeführte Nebenprodukte werden also beispielsweise zu 70 Vol.-% bis 80 Vol.-% dem Reformer und zu 20 Vol.-% bis 30 Vol.-% dem ersten Reaktor zugeführt, um den Bedarf für elektrische Energie gering zu halten.

In einer Ausgestaltung des Verfahrens ist der erste Reaktor ein RWGS - Reaktor. Mit RWGS wird "Reverse Water-Gas Shift Reaction" abgekürzt. Ein RWGS - Reaktor ist also ein Reaktor, in dem eine umgekehrte Wassergas - Shift - Reaktion durchgeführt wird, um aus Wasserstoff und Kohlendioxid ein Gemisch aus Wasserstoff, Kohlenmonoxid und Wasser bzw. Wasserdampf zu erzeugen.

In einer Ausgestaltung des Verfahrens ist der zweite Reaktor ein Reaktor zur Durchführung einer Fischer - Tropsch - Synthese. Im zweiten Reaktor kann daher ein Katalysator auf Basis von Cobalt (Co) oder Eisen (Fe) vorhanden sein, um aus Synthesegas Kohlenwasserstoffe zu erzeugen.

Vorteilhaft werden in den ersten Reaktor ein Gemisch aus externem Wasserstoff und externem Kohlendioxid so eingeleitet, dass das Verhältnis von Wasserstoff zu Kohlenmonoxid 1,6 bis 2,2, vorzugsweise 1,8 bis 2, beträgt, welches in den Reaktor zur Durchführung einer Fischer - Tropsch - Synthese eingeleitet wird.

Insbesondere wird der erste Reaktor so betrieben, dass Temperaturen von 800° bis 1000°C im ersten Reaktor erreicht werden, um die Bildung des Nebenprodukts CH₄ gering zu halten. Dafür benötigte Energie kann im Bedarfsfall vorteilhaft vom Reformer zum ersten Reaktor geleitet werden. Aus dem ersten Reaktor kann dann beispielsweise Synthesegas herausgeführt werden, welches anfangs 800°C bis 1000°C heiß ist.

Insbesondere wird der zweite Reaktor so betrieben, dass Temperaturen von 200° bis 250°C im zweiten Reaktor erreicht werden, um günstig Kohlenwasserstofffraktionen herstellen zu können.

Werden höhermolekulare Kohlenwasserstofffraktionen erzeugt, so werden diese vorzugsweise von der Trennstation einem Hydrocracker zugeführt, um zu flüssigen Kraftstoffen und zwar insbesondere zu Kerosin und Diesel zu gelangen. Im Hydrocracker gecrackte höhermolekularen Kohlenwasserstoffe werden anschließend beispielsweise einer Rektifikationskolonne zugeführt, um darüber gewünschte flüssige Kraftstoffe wie Kersosin und Diesel zu erhalten. Werden durch Rektifikation in der Rektifikationskolonne neben Kerosin und Diesel immer noch höhermolekulare Kohlenwasserstoffe im Vergleich zu Kerosin und Diesel erhalten, so werden diese vorzugsweise in den Hydrocracker zurückgeführt. Andere Nebenprodukte wie Benzinfraktionen oder Naphtha werden vorzugsweise dem Reformer zugeführt.

Der Reformer kann in einer Ausgestaltung exotherm gefahren werden, um für die Rektifikation benötigte Wärme vom Reformer zur Rektifikationskolonne zu leiten. Insbesondere wird der Reformer so gefahren, dass Temperaturen von 800° bis 1000°C erreicht werden.

Vorteilhaft gibt es eine Zuführung von dem zweiten Reaktor zur Rektifikationskolonne, um für die Rektifikation benötigte Wärme in Form von Wasserdampf von dem zweiten Reaktor kommend zur Rektifikationskolonne zu leiten.

Wasserdampf, der für die Reformierung benötigt wird, wird vorteilhaft dem ersten Reaktor entnommen und in den Reformer eingeleitet, also dem Reformer zugeführt.

Eine Vorrichtung zur Durchführung eines Verfahrens kann umfassen:
- einen ersten Reaktor,
- einen Einlass für ein Einleiten von Kohlendioxid und Wasserstoff in den ersten Reaktor;
- einen Katalysator in dem ersten Reaktor für eine katalytische Umwandlung eines Gemisches aus Kohlenmonoxid, Kohlendioxid und Wasserstoff in Methanol und/oder Dimethylether;
- einen Reformer;
- eine Verbindung zwischen dem ersten Reaktor und dem Reformer für ein Zuführen von Kohlenmonoxid aus dem Reformer in den ersten Reaktor;
- einen zweiten Reaktor;
- eine Verbindung zwischen dem ersten Reaktor und dem zweiten Reaktor für ein Zuführen von Methanol und/oder Dimethylether aus dem ersten Reaktor in den zweiten Reaktor;
- einen Katalysator in dem zweiten Reaktor für eine katalytische Umwandlung von Methanol und/oder Dimethylether in gasförmige und flüssige Kohlenwasserstoffe in dem zweiten Reaktor;
- eine Trennstation;
- eine Verbindung zwischen dem zweiten Reaktor und der Trennstation für ein Zuführen der gasförmigen und flüssigen Kohlenwasserstoffe in die Trennstation;
- eine Trenneinrichtung in der Trennstation, durch die flüssiger Kraftstoff in der Trennstation abgetrennt und durch die flüssiger Kraftstoff aus der Trennstation herausgeführt werden kann;
- eine Verbindung zwischen der Trennstation und dem Reformer, durch die ein Nebenprodukt aus der Trennstation dem Reformer zugeführt werden kann;
- eine Reformierungseinrichtung in dem Reformer, durch die aus dem Nebenprodukt Kohlenmonoxid erzeugt werden kann.

Die Verbindung zwischen dem zweiten Reaktor und der Trennstation für ein Zuführen der gasförmigen und flüssigen Kohlenwasserstoffe in die Trennstation kann einen weiteren Reaktor umfassen, in dem Dimethylether in gasförmige und flüssige Kohlenwasserstoffe umgewandelt wird.

Der erste Reaktor kann einen Gasauslass aufweisen. Es kann eine Steuereinrichtung vorhanden sein, durch die Gas aus dem Gasauslass periodisch herausgeführt werden kann.

Zwischen dem ersten und dem zweiten Reaktor kann eine weitere Trennstation vorhanden sein, um Methanol geeignet zu separieren. Als weitere Trennstation kann eine Rektifikationskolonne vorgesehen sein. Eine weitere Trennstation kann vorgesehen sein, um das im zweiten Reaktor entstandene Wasser von den Kohlenwasserstoffen zu trennen. Dafür kann ebenfalls eine Rektifikationskolonne vorgesehen sein.

Durch die Erfindung können flüssige Kraftstoffe deutlich effizienter hergestellt werden. Der dafür benötigte Mehraufwand kann überraschend gering gehalten werden. Auch wirkt sich die Erfindung positiv auf die Lebensdauer von Komponenten aus. Durch ein hohes Kohlenmonoxid zu Wasserstoffverhältnis im Produktstrom des Reformers und anschließender Beimischung zum Produktstrom des RWGS - Reaktors sowie Einstellung des für die Fischer-Tropsch- Synthese günstigen Wasserstoff zu Kohlenmonoxid Verhältnisses mittels Wasserstoffzugabe wird die thermische Beheizung für den RWGS - Reaktor gering gehalten.

Nachfolgend wird die Erfindung anhand von Ausführungsbeispielen näher erläutert. Es zeigen:
- Figur 1:: nicht erfindungsgemäße Vorrichtung für eine Herstellung von Benzin;
- Figur 2:: erfindungsgemäße Vorrichtung für eine Herstellung von Benzin;
- Figur 3:: Ergebnisse;
- Figur 4:: erfindungsgemäße Vorrichtung für eine Herstellung von Kerosin sowie Diesel;
- Figur 5:: Blockfließbild des entwickelten Fischer-Tropsch-Verfahrens;
- Figur 6:: Blockfließbild mit einem weiteren Reaktor;
- Figur 7:: Blockfließbild umfassend die Herstellung von Dimethylether.

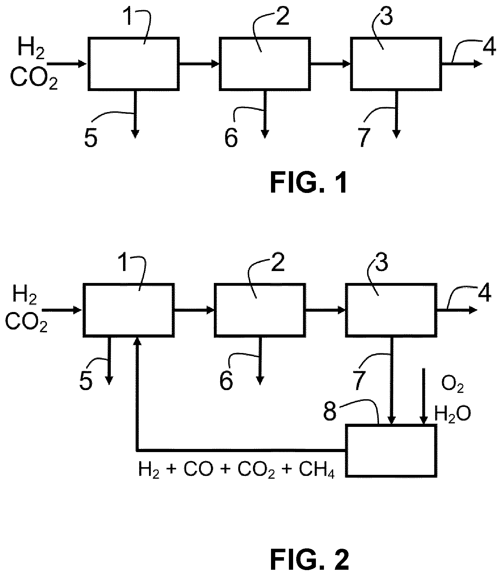

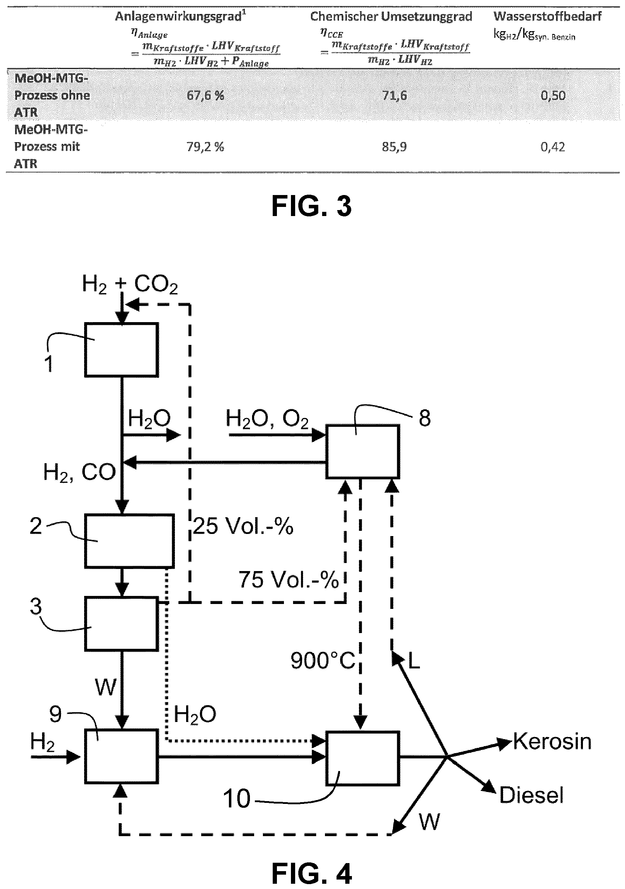

Es folgt die Beschreibung von zwei Simulationsbeispielen mit anschließendem Vergleich. Das erste Beispiel ist ein Vergleichsbeispiel welches anhand der Figur 1 erläutert wird. Das zweite erfindungsgemäße Beispiel wird anhand der Figur 2 erläutert. Ergebnisse sind in der Figur 3 gezeigt. Das anhand der Figur 2 erläuterte Beispiel dient der Herstellung von Benzin.

Die Figur 1 zeigt eine Vorrichtung mit einem ersten Reaktor 1, einem zweiten Reaktor 2 und einer Trennstation 3. Wie in der Figur 1 angedeutet, wird ein Gemisch aus Wasserstoff und Kohlendioxid in den ersten Reaktor 1 eingeleitet. Im ersten Reaktor wird mithilfe eines Katalysators das aus Wasserstoff und Kohlendioxid bestehende Gemisch katalytisch in Methanol umgewandelt. Es entsteht dabei Wasser bzw. Wasserdampf als Koppelprodukt. Aus dem ersten Reaktor 1 wird das Methanol herausgeleitet und in den zweiten Reaktor 2 hineingeleitet. Außerdem werden im ersten Reaktor 1 entstandene Koppelprodukte aus dem ersten Reaktor 1 herausgeführt und zwar über den Auslass 5.

Im zweiten Reaktor 2 wird Methanol katalytisch in Kohlenstoffwasserstoffe umgewandelt. Wasser bzw. Wasserdampf entsteht dabei als Koppelprodukt. Koppelprodukte werden über den Auslass 6 herausgeleitet. Die Kohlenwasserstoffe werden aus dem zweiten Reaktor in die Trennstation 3 eingeleitet. Flüssiger Kraftstoff wird über den Auslass 4 aus der Trennstation herausgeleitet. Nebenprodukte werden aus dem Auslass 7 aus der Trennstation herausgeleitet.

Die in der Figur 2 gezeigte Vorrichtung umfasst im Vergleich zu der in der Figur 1 gezeigten Vorrichtung zusätzlich einen Reformer 8. Die aus der Trennstation herausgeführten Nebenprodukte 7 werden in den Reformer 8 eingeleitet. In den Reformer werden außerdem Sauerstoff (O₂) und Wasserdampf (H₂O) eingeleitet. Als Nebenprodukt anfallende kürzere Kohlenwasserstoffe werden in dem Reformer unter Einsatz von Sauerstoff und Wasserdampf in Synthesegas zerlegt. Der eingeleitete Wasserdampf kann dem ersten und/oder dem zweiten Reaktor 1, 2 entnommen worden sein.

Nach Durchführung der Reformierung wird aus dem Reformer 8 ein aus Wasserstoff, Kohlenmonoxid, Kohlendioxid und Methan bestehendes Gemisch herausgeleitet und in den ersten Reaktor 1 eingeleitet.

Der Prozess zur Methanolsynthese wurde wie folgt ausgelegt.
- Reaktor: 250 °C, 80 bar.
- Vorwärmung des Eduktstroms des Reaktors mit dem Produktstrom des Reaktors
- Abtrennung nicht umgesetzter Gase durch Drosselung und Abkühlung des Produktstroms auf 80 °C und 75 bar
- Rückführung der Gase zum Reaktor
- Rohmethanol (Methanol+Wasser) wird in einer Kolonne bei 1 bar aufgetrennt.
- im Rohmethanol (Methanol+Wasser) ist noch CO₂ gelöst. Der Gehalt kann z.B. nach der Rektifikation durch weitere Abkühlung verringert werden.

Zur technischen Umsetzung werden normalerweise werden mehrere Kolonnen verwendet, da mit Nebenprodukten, wie Ethanol und Ameisensäure, gerechnet wird.

Es wurde davon ausgegangen, dass sich die Produktzusammensetzung aus der Lage des Gleichgewichts durch Minimierung der freien Gibbs-Energie ergibt. Gewählt wurde für diesen Fall ein molares H₂:CO/CO₂ Verhältnis von 4 (gemäß Druckschrift Otto, A., Chemische, verfahrenstechnische und ökonomische Bewertung von Kohlendioxid als Rohstoff in der chemischen Industrie; 2015, Forschungszentrum Jülich GmbH: Jülich).

Es wurde angenommen, dass neben dem Koppelprodukt Wasser keine Nebenprodukte entstehen. Innerhalb des Teilprozesses wurden nicht umgesetzte Edukte rückgeführt. Nach Produktseparation mittels Rektifikationskolonne hat das Produkt Methanol eine Reinheit von 99,9 Gew.-% und entspricht damit dem AA-Grade. Zur Steigerung des Prozesswirkungsgrades wurde die Reaktorabwärme im Verdampfer der Kolonne genutzt.

Der MTG-Prozess wurde basierend auf dem NREL-Report von Phillips, S.D., et al., Gasoline from Wood via integrated Gasification, Synthesis, and Methanol-to-Gasoline Technologies. 2011, NREL - National Renewable Energy Laboratory: Golden, Colorado; p. 115 ausgelegt. Der Bericht liefert eine für die Simulation hinreichend genaue Produktverteilung eines Wirbelschichtreaktors. In Übereinstimmung mit der vorgenannten Druckschrift von Phillips et al. wird von einem 100%igen Methanolumsatz ausgegangen.

Die Produktaufbereitung (De-Ethanisierung, Alkylierung, ...) wird vernachlässigt, sodass der Prozess ähnlich zu der in Wesseling [siehe z.B. Druckschrift Avidan, A.A., Gasoline and Distillate Fuels From Methanol, in Studies in Surface Science and Catalysis, D.M. Bibby, et al., Editors. 1988, Elsevier. p. 307-323 DOI: https:// doi.org/10.1016/S0167-2991(09)60524-3.) von 1982-1985 betriebenen Demonstrationsanlage ist.

Wie in Figur 2 gezeigt, werden die Produkte des Methanol-to-Gasoline-Prozesses, die nicht direkt zur Benzinfraktion gehören und auch nicht mit Hilfe von Reaktoren (z.B. De-Ethanizer, Stabilizer, Alkylation-Reaktoren) weiter in synthetisches Benzin umgewandelt werden können, nach einer Abtrennung zum autothermischen Reformer (abgekürzt: ATR) geleitet und dort (unter anderem) wieder in Eduktgase für die Methanolsynthese umgewandelt. Die Produkte des autothermischen Reformers werden als Edukte der Methanolsynthese verwendet.

Für die Simulation des autothermischen Reformers wurde das folgende beispielhafte Design gewählt:
Die Produktverteilung wird mittels des thermodynamischen Gleichgewichts (errechnet durch Minimierung der Gibbs-Energie) für eine Reaktoraustrittstemperatur von 900 °C bestimmt. Diese Temperatur kann auch höher oder niedriger sein. Eine höhere Temperatur begünstigt die Lage des thermodynamischen Gleichgewichts, ist aber apparatetechnisch schwieriger umzusetzen. Der Edukstrom wird mit dem Produktstrom vorgeheizt. Dieser Schritt dient zur Wärmerückgewinnung und ist nicht zwingend notwendig. Für die Reformierungsreaktion ist die Zugabe von Wasser (in den Edukstrom oder den Reaktor] wichtig. Hier wurde beispielhaft ein H₂O/C-Verhältnis von 0,6 gewählt. Dieses Verhältnis kann variiert werden. Die O₂-Zugabe in den Reaktor wurde so geregelt, dass der Reaktor autotherm ist.

Der Reaktor könnte auch exotherm oder endotherm sein. Bei der endothermen Fahrweise kann die Wärmebereitstellung durch eine elektrische Aufheizung des Eduktstroms, beispielsweise auf 1000 °C, erfolgen. Der Sauerstoff muss nicht in Reinform zugegeben werden. Beispielsweise wäre auch Luft oder ein O₂/H₂O-Gemisch möglich. Wie die Reaktortemperatur ist auch der Reaktordruck variabel und beeinflusst das thermodynamische Gleichgewicht der Reaktion.

Der Vergleich der Simulationen eines Ausführungsbeispiels mit und ohne autothermen Reformer ergibt eine Steigerung des Anlagenwirkungsgrades und des chemischen Umsetzungsgrads wie aus der Figur 3 ersichtlich. Es wird weniger Wasserstoff für die Produktion benötigt.

In der Simulation wird der Reaktoraustrittsstrom aus dem zweiten Reaktor heraus in eine Gas- und eine Benzinfraktion aufgeteilt. Dabei geht nach Wasserabscheidung etwa 25 Gew.-% in die Gas- und 75 Gew.-% in die Benzinfraktion. Der hohe Gasanteil liegt an den etwas zu hohen Pentan und Isobutananteilen im Gas. Die Benzinfraktion erfüllt allerdings weitestgehend die Standards für konventionelles Benzin.

Versuche haben gezeigt, dass sich durch die Erfindung die Lebensdauer von eingesetzten Katalysatoren verbessern lässt und zwar zumindest von CuO/ZnO/Al₂O₃-Katalysatoren.

Mit dem gleichen Konzept (Rückverwertung der Nebenprodukte aus der Kohlenwasserstoffsynthese durch Reformierung und anschließende Verwertung der Reformierungsprodukte bei der Synthese des Vorprodukts zu den Kohlenwasserstoffen) ist auch Folgendes umsetzbar:
- Variation des Zwischenprodukts:
   ∘ Bei der Reaktion von Methanol zu Kohlenwasserstoffen wird im Reaktor in der Regel das Zwischenprodukt DME gebildet.
   ∘ Man kann auch Dimethylether (DME) anstelle von Methanol produzieren und dieses anschließend zu Kohlenwasserstoffen umwandeln.
   ∘ DME kann man aus H₂/CO, H₂/CO/CO₂, H₂/CO₂ oder konventionell aus Methanol herstellen.
   ∘ Es kann Methanol zunächst zu DME umgewandelt und anschließend in einem nächsten Reaktor zu Kohlenwasserstoffen umgewandelt werden. Damit kann Einfluss auf die Produktverteilung im Reaktor genommen werden.
- Variation der Kohlenwasserstoffsynthese

Anstelle des MTG-Verfahrens zur Synthese von Kohlenwasserstoffen, die der Benzinfraktion zugeordnet werden können, können mit dem MTO (Methanol-to-Olefins) oder DTO (DME-to-Olefins) auch Olefine gebildet werden. Auch hier ist die Bildung der gewünschten Olefine nicht selektiv und es werden stets Nebenprodukte gebildet. Olefine sind wichtige Basischemikalien und eignen sich auch zur Synthese von Diesel und Kerosin. Derzeit werden Olefine hauptsächlich aus Erdöl gewonnen.

Die Figur 4 zeigt ein Ausführungsbeispiel zur Herstellung von Kerosin sowie Diesel. Beispielweise durch Elektrolyse hergestellter Wasserstoff und Kohlendioxid werden in einen ersten Rektor 1 eingeleitet. Der erste Reaktor 1 ist ein RWGS - Reaktor. Im RWGS - Reaktor 1 wird katalytisch durch eine umgekehrte Wassergas - Shift Reaktion ein Gemisch Wasserstoff, Kohlenmonoxid und Wasserdampf erzeugt. Das aus dem ersten Reaktor 1 herausgeführte Synthesegas, also der Wasserstoff und das Kohlenmonoxid, werden in einen zweiten Reaktor 2 eingeleitet. Im zweiten Reaktor findet eine Fischer - Tropsch - Synthese statt. Das Synthesegas wird also im zweiten Reaktor 2 heterogenkatalytisch in ein Spektrum gasförmiger und flüssiger Kohlenwasserstoffe umgewandelt. Die im zweiten Reaktor 2 erzeugten Kohlenwasserstoffe werden einer Trennstation 3 zugeführt. In der Trennstation 3 werden höhermolekulare Kohlenwasserstofffraktionen W (Wachse, Paraffin), die für die Herstellung von Kerosin und Diesel geeignet sind, für eine Weiterverarbeitung einem Hydrocracker 9 zugeführt. Mit Hydrocracker ist ein Reaktor gemeint, in dem ein katalytisches Crackverfahren in Gegenwart von Wasserstoff durchgeführt wird, um die höhermolekularen Kohlenwasserstofffraktionen in Zwischenprodukte zur Herstellung von flüssigen Kraftstoffen umzuwandeln. Für ein Cracken kann das Einleiten von Wasserstoff in den Hydrocracker vorgesehen sein. Die Zwischenprodukte werden einer Rektifikationskolonne 10 zugeführt. Mittels der Rektifikationskolonne 10 werden aus den Zwischenprodukten Kerosin K und Diesel D erhalten. Höhermolekulare Kohlenwasserstoffe W werden wieder dem Hydrocracker 9 zugeführt. Niedermolekulare Kohlenwasserstoffe L wie Benzin werden dem Reformer 8 zugeführt.

Der Reformer 8 kann exotherm betrieben werden. Es können so Temperaturen von beispielsweise 900 °C erzeugt werden. Die so erzeugte Wärme wird der Rektifikationskolonne 10 zugeführt, um Kerosin und Diesel zu gewinnen. Dampf zum Betrieb der Rektifikationskolonne 10 (oder Destillationskolonne) kann durch Kühlung des Reaktors 2 erzeugt werden

Der Reformer 8 kann exotherm gefahren werden, sodass die Abwärme im Verdampfer der Destillationskolonne verwendet werden kann. Somit entfällt die normalerweise notwendige Feuerung.

Vom Reformer 8 sowie vom Reaktor 2 gibt es keinen direkten Stoffstrom zur Rektifikationskolonne 10.

Sauerstoff, der dem Reformer 8 für die Durchführung zugeführt wird, kann Sauerstoff sein, der durch Elektrolyse extern erzeugt wurde. Damit kann nicht nur der durch Elektrolyse erzeugte externe Wasserstoff verwendet werden, sondern außerdem der dann erzeugte externe Sauerstoff.

Nebenprodukte werden von der Trennstation 3 zu ca. 25 Vol.-% in den ersten Reaktor 1 eingeleitet und zu 75 Vol.-% in den Reformer 8. Im Reformer 8 hergestellte Produkte, die Kohlenmonoxid umfassen, werden dem zweiten Reaktor 2 zugeführt.

Das Verhältnis von Wasserstoff zu Kohlendioxid, welches extern bereitgestellt dem ersten Reaktor zugeführt wird, ist so gewählt, dass das Verhältnis von Wasserstoff zu Kohlenmonoxid, welches in den zweiten Reaktor 2 gelangt, in etwa zwei beträgt.

Für die Durchführung einer Reformierung werden in den Reformer 8 Sauerstoff und Wasserdampf eingeleitet. Der in den Reformer 8 eingeleitete Wasserdampf kann aus dem ersten Reaktor 1 zugeführt worden sein.

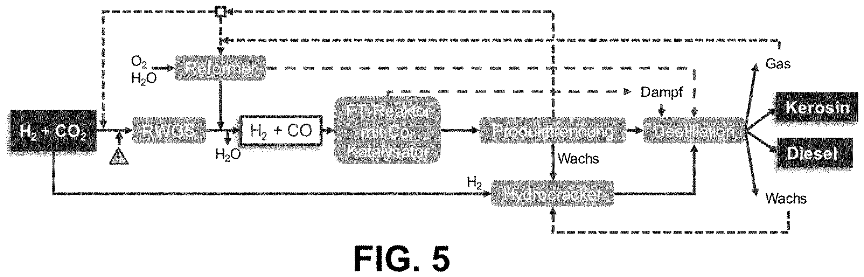

Figur 5 zeigt ein Blockfließbild des im Rahmen dieser Erfindung entwickelten Konzepts für ein Fischer-Tropsch-Verfahren. Zur Maximierung des Gehalts an Mitteldestillaten wurde das Niedertemperatur-FT-Verfahren mit Co-Katalysatoren gewählt (FT = Fischer - Tropsch).

Das in Figur 5 dargestellte Verfahren setzt sich aus einem RWGS-Reaktor, einem Reformer, einem FT-Reaktor, einem Hydrocracker und einer atmosphärischen Destillationskolonne zusammen. Reale Blasensäulenreaktoren haben sowohl einen gasförmigen als auch einen flüssigen Reaktoraustrittsstrom. Da das angewendete RStoic-Modell zur Simulation des FT-Reaktors nur einen Austrittstrom hat, wird dieser im direkten Anschluss isobar und isotherm die Gas- und Flüssigphase aufgetrennt. Nach Abtrennung der wässrigen Phase wird die Flüssigphase, die zu 81 Gew.-% aus C₁₈₊-Kohlenwasserstoffen besteht, zum Hydrocracker geleitet. Die weitere Produkttrennung direkt hinter dem FT-Reaktor erfolgt schrittweise isobare Kondensation durch Abkühlung. In Dekantern wird dabei die unterschiedliche Dichte sowie Polarität der Kohlenwasserstoffe gegenüber Wasser genutzt, um letzteres abzutrennen. Die erste Abkühlung ist auf 80 °C, dessen Flüssigphase in der Literatur als "hot condensate" bezeichnet wird. Zur Vermeidung von Feststoffausfall wird die Temperatur der wachsreichen Stoffströme immer über 70 °C gehalten. Die Kondensatströme der ersten und zweiten Abkühlungsstufen enthalten kaum C₁₈₊Kohlenwasserstoffe und werden nach Wasserabscheidung direkt zur Destillation geleitet.

Im Fall des in Figur 5 dargestellten Verfahrens erfolgt die Synthesegasbereitstellung für den FT-Reaktor mit Hilfe eines RWGS-Reaktors sowie eines Reformers. Insbesondere der Einsatz des Reformers zur Rückverwertung der C-Fraktionen, die zu kurz sind, um der Kerosinfraktion zugeordnet zu werden, ist eine Innovation. Im Reformer werden diese C-Fraktionen unter Einsatz von O₂ und H₂O zu Synthesegas (H₂/CO) reformiert. Die Rückführung der durch Abkühlung aus dem FT-Produktstrom abgetrennten kurzen Kohlenwasserstoffe zum RWGS-Reaktor ist typisch für FT-Verfahren mit Co-Katalysatoren. Bei dem in der Figur 5 dargestellten Verfahren werden diese Gase teilweise zum Reformer geleitet, was die Beheizung des RWGS-Reaktors entlastet. Um Feuerung zu vermeiden, wird der Eduktstrom des RWGS-Reaktors elektrisch auf maximal 1000 °C beheizt.

Die Produkte des RWGS-Reaktors und des Reformers werden zusammengeführt und dem FT-Reaktor zugeführt. Die Einstellung des exakten H₂/CO-Verhältnisses im Eduktstrom des FT-Reaktors erfolgt über die Einstellung des H₂/CO₂-Verhältnisses im Eduktstrom für die Gesamtanlage.

Die Wasserabtrennung zwischen RWGS- und FT-Reaktor ist eigentlich typisch für Prozesse mit wasseranfälligen Fe-Katalysatoren, dient dagegen hier zur Verringerung des Volumens des FT-Reaktors. Der Reformer wird nicht autotherm, sondern exotherm gefahren, um die thermische Leistung, die der Verdampfer des Destillationskolonne benötigt, bereitzustellen (siehe Figur 5, gestrichelter Pfeil vom Reformer zur Destillationskolonne). Durch zusätzliche Verwertung der destillativ abgetrennten Wachse im Hydrocracker sind paraffinisches Kerosin und paraffinischer Diesel die einzigen Produkte des Verfahrens.

Insgesamt gelingt so die Herstellung von Kerosin und Diesel aus Kohlendioxid und Wasserstoff mit geringem energetischem Aufwand auf eine für eingesetzte Katalysatoren schonende Weise.

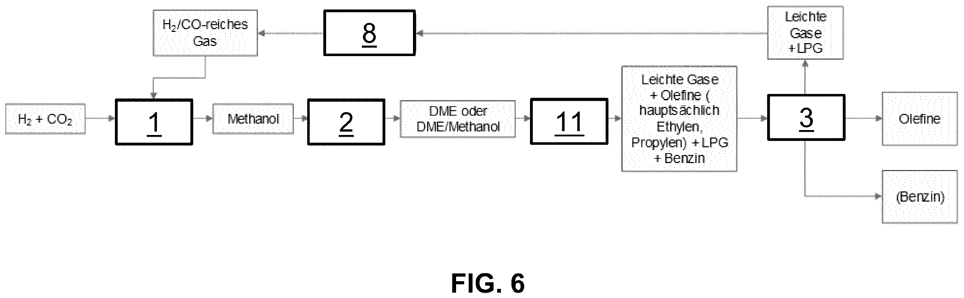

In der Figur 6 wird der Fall dargestellt, dass im zweiten Reaktor 2 zunächst Dimethylether (DME) oder ein Gemisch aus Dimethylether und Methanol erzeugt wird. In einem weiteren Reaktor 11 werden in einem nachfolgenden Verarbeitungsschritt Kohlenwasserstoffe aus dem Dimethylether hergestellt. Die im weiteren Reaktor 11 erzeugten Kohlenwasserstoffe werden dann der Trennstation 3 zugeführt. Aus den Olefinen können dann beispielsweise flüssige Kraftstoffe hergestellt werden.

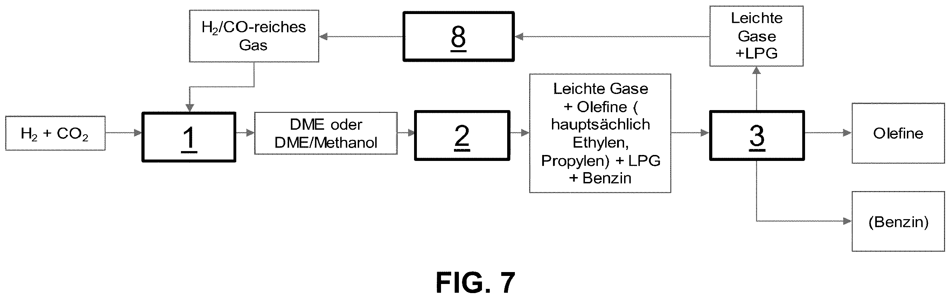

In der Figur 7 wird der Fall dargestellt, dass im ersten Reaktor 1 Dimethylether oder ein Gemisch aus Dimethylether und Methanol hergestellt wird.

## Patentansprüche

1. Verfahren zur Herstellung von flüssigem Kraftstoff mit den Schritten:
• Einleiten von Kohlendioxid und Wasserstoff in einen ersten Reaktor (1);
• Zuführen von Kohlenmonoxid aus einem Reformer (8) in den ersten Reaktor (1);
• Herstellen von Methanol aus dem Wasserstoff, Kohlendioxid und Kohlenmonoxid in dem ersten Reaktor (1) mithilfe eines Katalysators und/oder Herstellen von Synthesegas in dem ersten Reaktor (1) und/oder Herstellen von Dimethylether in dem ersten Reaktor (1);
• Zuführen von Methanol und/oder Synthesegas und/oder Dimethylether aus dem ersten Reaktor (1) in einen zweiten Reaktor (2);
• Herstellen von Kohlenwasserstoffen aus dem Methanol und/oder dem Synthesegas und/oder dem Dimethylether;
• Zuführen der Kohlenwasserstoffe in eine Trennstation (3);
• Abtrennen von flüssigem Kraftstoff in der Trennstation (3) und Herausführen des abgetrennten flüssigen Kraftstoffs aus der Trennstation (3);
• Zuführen eines Nebenprodukts aus der Trennstation (3) in den Reformer (8);
• Herstellen des Kohlenmonoxids in dem Reformer (8) aus dem Nebenprodukt.

2. Verfahren nach Anspruch 1, **dadurch gekennzeichnet, dass** aus dem Nebenprodukt mit Hilfe des Reformers (8) unter Einsatz von O₂ und H₂O das Kohlenmonoxid hergestellt wird.

3. Verfahren nach dem vorhergehenden Anspruch, **dadurch gekennzeichnet, dass** durch autotherme Reformierung das Kohlenmonoxid aus dem Nebenprodukt hergestellt wird.

4. Verfahren nach einem der vorhergehenden Ansprüche, **dadurch gekennzeichnet, dass** aus dem Reformer (8) H₂, CO, und/oder CO₂ herausgeleitet und in den ersten Reaktor (1) hineingeleitet wird.

5. Verfahren nach einem der vorhergehenden Ansprüche, **dadurch gekennzeichnet, dass** Wasserdampf, der in dem ersten oder zweiten Reaktor (1, 2) entsteht, in die Trennstation (3) für die Durchführung der Trennung und/oder in den Reformer (8) für die Durchführung der Reformierung eingeleitet wird.

6. Verfahren nach einem der vorhergehenden Ansprüche, **dadurch gekennzeichnet, dass** aus dem ersten Reaktor (1) periodisch Gas herausgeführt wird.

7. Verfahren nach einem der vorhergehenden Ansprüche, **dadurch gekennzeichnet, dass** Wasserstoff durch Elektrolyse erzeugt wird und der durch Elektrolyse erzeugte Wasserstoff in den ersten Reaktor (1) eingeleitet wird und/oder dass Kohlendioxid durch Abscheidung auf Luft erhalten wird und das durch Abscheidung erhaltene Kohlendioxid in den ersten Reaktor (1) eingeleitet wird.

8. Verfahren nach einem der vorhergehenden Ansprüche, **dadurch gekennzeichnet, dass** in den zweiten Reaktor Wasserstoff im Verhältnis von 1,6 bis 2,2 eingeleitet wird.

9. Vorrichtung zur Durchführung eines Verfahrens nach einem der vorhergehenden Ansprüche mit:
• einem ersten Reaktor (1),
• einem Einlass für ein Einleiten von Kohlendioxid und Wasserstoff in den ersten Reaktor (1);
• einem Katalysator in dem ersten Reaktor (1) für eine katalytische Umwandlung des Gemisches aus Kohlenmonoxid, Kohlendioxid und Wasserstoff in Methanol;
• einem Reformer (8);
• einer Verbindung zwischen dem ersten Reaktor (1) und dem Reformer (8) für ein Zuführen von Kohlenmonoxid aus dem Reformer (8) in den ersten Reaktor (1);
• einem zweiten Reaktor (2);
• einer Verbindung zwischen dem ersten Reaktor (1) und dem zweiten Reaktor (2) für ein Zuführen von Methanol aus dem ersten Reaktor (1) in den zweiten Reaktor (2);
• einem Katalysator in dem zweiten Reaktor (2) für eine katalytische Umwandlung von Methanol in gasförmige und flüssige Kohlenwasserstoffe in dem zweiten Reaktor (2) oder in Dimethylether;
• einer Trennstation (3);
• einer Verbindung zwischen dem zweiten Reaktor (2) und der Trennstation (3) für ein Zuführen von gasförmigen und flüssigen Kohlenwasserstoffen in die Trennstation (3);
• einer Trenneinrichtung in der Trennstation (3), durch die flüssiger Kraftstoff in der Trennstation (3) abgetrennt und durch die flüssiger Kraftstoff aus der Trennstation (3) herausgeführt werden kann;
• einer Verbindung zwischen der Trennstation (3) und dem Reformer (8), durch die ein Nebenprodukt aus der Trennstation dem Reformer (8) zugeführt werden kann;
• einer Reformierungseinrichtung in dem Reformer (8), durch die aus dem Nebenprodukt Kohlenmonoxid erzeugt werden kann.

10. Vorrichtung nach dem vorhergehenden Anspruch, **dadurch gekennzeichnet, dass** der erste Reaktor (1) einen Gasauslass aufweist und dass eine Steuereinrichtung vorhanden ist, durch die Gas aus dem Gasauslass periodisch herausgeführt werden kann.

11. Vorrichtung nach einem der drei vorhergehenden Ansprüche, **dadurch gekennzeichnet, dass** eine Verbindung zwischen der Trennstation (3) und dem ersten Reaktor (1) vorhanden ist, durch die ein Nebenprodukt aus der Trennstation (3) dem ersten Reaktor (1) zugeführt werden kann.

12. Vorrichtung nach dem vorhergehenden Anspruch, **gekennzeichnet durch** eine Steuereinrichtung, die bewirkt, dass die Nebenprodukte zu einem überwiegenden Teil dem Reformer und zu einem geringeren Teil dem ersten Reaktor zugeführt werden.

13. Vorrichtung nach einem der fünf vorhergehenden Ansprüche, **dadurch gekennzeichnet, dass** der erste Reaktor ein RWGS - Reaktor und der zweite Reaktor ein Reaktor zur Durchführung einer Fischer-Tropsch-Synthese ist.

14. Vorrichtung nach einem der sechs vorhergehenden Ansprüche, **dadurch gekennzeichnet, dass** eine Zuführungseinrichtung vorhanden ist, über die Wasserdampf von dem ersten und/oder zweiten Reaktor (1, 2) ein oder mehreren Rektifikationskolonnen (3, 10) und/oder dem Reformer (8) zugeführt werden kann.

15. Vorrichtung nach einem der sieben vorhergehenden Ansprüche, **dadurch gekennzeichnet, dass** eine Wärmetransporteinrichtung vorhanden ist, mit der Wärme von dem Reformer (8) ein oder mehreren Rektifikationskolonnen (3, 10) und/oder dem ersten Reaktor zugeführt werden kann.
